# EUROPEAN PATENT APPLICATION

(11) **EP 0 908 190 A2**
(43) Date of publication of application: **14.04.1999**
(21) Application number: 98203018.1
(22) Date of filing: 10.09.1998
(51) Int. Cl.: A61L 11/00

(54) **Waste sterilisation**

(30) Priority: 09.10.1997 GB 9721290
(71) Applicant: General Waste Reduction Limited, London SE1 1HH (GB)
(72) Inventor: Clarke, Howard Morgan, Liss, Hampshire GU33 7NT (GB)
(74) Representative: Brooks, Nigel Samuel

(57) **Abstract**

A method and apparatus therefor, for the sterilisation of waste material, particularly though not exclusively medical waste is provided. The waste material is comminuted, loaded into a pressure, vessel, treated with steam and removed from the pressure vessel. The latent heat in the steam heats the waste material to a sufficiently high temperature to effect sterilisation. The pressure vessel is purged of air prior to being pressurised with steam. After sterilisation the water that has built up in the pressure vessel is drained off, before the pressure in the vessel is released. As the vessel and its contents are still at a high temperature, this sudden release in pressure results in a considerable drying of the waste material.

## Description

The present invention relates to waste sterilisation apparatus, particularly though not exclusively, suitable for sterilising hazardous waste. Often the waste will be medical waste.

Medical waste has typically been disposed of in incinerators. However, few of these incinerators meet the new emissions standards and other ways have to be found of disposing of this waste. Household waste is often disposed of in landfill sites, but due to potentially hazardous material in medical waste, the material has to be sterilised in some way. One method that has been used is heating the material directly on a hot surface. This is a very inefficient and time-consuming method of sterilising the material. Another method that has been used is to treat the material with chemicals to destroy the contamination. The product then contains a lot of equally hazardous chemicals, which need treating before being disposed of safely.

This type of waste material typically comprises mostly plastics material, paper and cotton. The plastics material comprises packaging and disposable articles, for example, syringes, blood transfusion kits, tubes and gloves, and the paper and cotton material includes tissues, cotton wool and bandages. Glass vials and needles are normally disposed of separately but occasionally will be placed in the general waste material.

The object of the present invention is to provide a method and apparatus for the sterilisation of waste material, particularly material comprising mostly manufactured material such as paper and plastics goods together with some hazardous material.

According to the present invention there is provided a method of sterilising waste material consisting in the steps of: -
- comminuting the waste material;
- loading the comminuted waste material into a pressure vessel and closing the vessel;
- applying steam to the material in the pressure vessel at least until the steam pressure in the vessel is substantially steady, whereby the material is heated to elevated temperature and sterilised; and
- removing the sterilised waste material from the pressure vessel.

The application of the steam to the waste material causes condensation of the steam on the material and walls of the pressure vessel so that they are heated by the latent heat released. The steam continues to enter the vessel whilst the condensation is occurring. This ceases at least at the material when the latter has reached the saturation temperature for the steam pressure in the vessel, i.e. when it has been heated to sterilisation temperature. Normally the steam will continue to be applied until a sufficient time has elapsed to effect sterilisation at the given temperature.

Preferably the air is substantially eliminated from the pressure vessel before it is pressurised with steam. This can be achieved by flowing the steam through the pressure vessel. As the steam enters the pressure vessel the air is expelled. Preferably the steam enters the pressure vessel through it's base and the air is allowed to escape through it's lid. Once all the air has been removed the pressure vessel can be sealed at the top.

Preferably the steam is applied by ducting it from a steam generator. The pressure of the steam is controlled at the steam generator. The pressure in the steam generator and the pressure vessel are allowed to reach equilibrium.

Preferably the steam is generated at a pressure of at least 0.8 bar (corresponding to a temperature of 116°C) and the predetermined period is at least substantially 30 minutes.

Preferably the pressure of the steam is at least 1.8 bar (corresponding to a temperature of 131.2°C) and the predetermined period is at least substantially 10 minutes.

Preferably the pressure is at least 4 bar (corresponding to a temperate of 151.8°C) and the predetermined period is at substantially 5 minutes least.

At the end of the sterilisation period the waste material will be saturated with water, and a quantity of water will have built up in the pressure vessel. Preferably, the steam is then isolated and most of this water is bled out of the pressure vessel before the steam pressure is released. The pressure vessel and its contents will still be hot and the sudden drop in pressure will result in the water left in the pressure vessel including that in the waste material being above its boiling point for the new lower pressure. Much of this water will therefore evaporate and there will be considerable drying of the waste material.

The material can then be removed from the pressure vessel. Whilst it is envisaged that the material can be cooled for safe disposal in a landfill site or otherwise, preferably the material will be pressed into briquettes or pellets. The high level of plastics in the material allows the material to be pelletised without the addition of any adhesive. The material will also have a high calorific value due to the plastics and paper content, which makes it suitable for burning in a furnace or boiler.

According to a second aspect of the present invention there is provided apparatus for use in the sterilisation of waste material, the apparatus comprising in combination: -
- comminution means, for preliminarily comminuting the waste material;
- a pressure vessel for receiving the comminuted waste material to be sterilised, the pressure vessel having:
   - a loading aperture via which the comminuted material can be received into the vessel;
   - a lid for pressure tightly closing the aperture; and
   - a steam inlet to the vessel;
- a chassis in which the comminution means and the pressure vessel are mounted;
- a steam generator for supplying steam at a predetermined pressure to the pressure vessel via the steam inlet for sterilising the waste material by application of steam to it.

Whilst it is envisaged that the steam inlet could be in the lid of the pressure vessel, preferably the steam inlet is in the base of the pressure vessel for allowing the steam to percolate upwards through the comminuted waste.

Preferably a perforated cone is provided at the steam inlet, for distributing application of steam to the comminuted waste.

Preferably the pressure vessel is pivotally mounted on the chassis to enable the pressure vessel to be inverted for waste discharge therefrom.

Preferably the lid is pivotally mounted to the chassis independently from the pressure vessel's pivot to enable the pressure vessel to be inverted while its lid is held in its open position.

Preferably the comminution means includes a shredder for shredding sheet material in the waste, a granulator for granulating particulate material in the waste, and a sieve to ensure that waste material with a major diameter greater than a predetermined value is retained in the granulator.

Preferably the apparatus includes an enclosure on the chassis for the comminution means, and a pump to maintain the enclosure below ambient pressure during comminution.

Preferably the pump is connected to a cyclone filter and a HEPA filter to remove any airborne contamination before venting.

Preferably the apparatus includes a screw conveyer to convey the comminuted waste material from the comminution enclosure to the pressure vessel.

Normally the pressure vessel will be purged of air prior to being pressurised with steam. Preferably the apparatus includes a first bubbler connected to a first outlet in the lid of the pressure vessel, such that as the pressure vessel is filled with steam expelled air bubbles through the bubbler.

Preferably the apparatus includes means for measuring the flow rate of air out of the first bubbler.

Preferably wherein the apparatus includes a second bubbler connected to a second outlet in the pressure vessel, such that as the pressure is released after sterilisation process, the steam is vented into the bubbler.

Whilst it is envisaged that the material could be cooled for disposal in a land fill site, preferably the apparatus includes a pelletiser for pressing the sterilised waste material into pellets.

To help understanding of the invention, a specific embodiment thereof will now be described with reference to the accompanying drawings, in which:
Figure 1 is a cross-sectional view of a container holding an apparatus according to the invention;
Figure 2 is a cross sectional view of the apparatus;
Figure 3 is a cross-sectional view of the comminution means of the invention;
Figure 4 is a cross-sectional view of the cyclone and filter system of the invention;
Figure 5 is a cross-sectional view of the pressure vessel of the apparatus.

The waste material produced by hospital wards and the like, includes plastic material, for example from packaging, syringes, tubes, and gloves; fibrous material, for example cotton bandages, and cotton wool; paper, for example card board boxes, tissues and newspapers; a small amount of metal from needles and bottle caps; a small quantity of glass for example glass vials; and miscellaneous other waste given to or used by patients, such flowers and food. Normally glass and needles should be disposed of separately in a "sharps" box, but occasionally some of this material may be placed with the general waste material. Inside the hospital the waste material is placed in yellow plastic bags, ready for disposal.

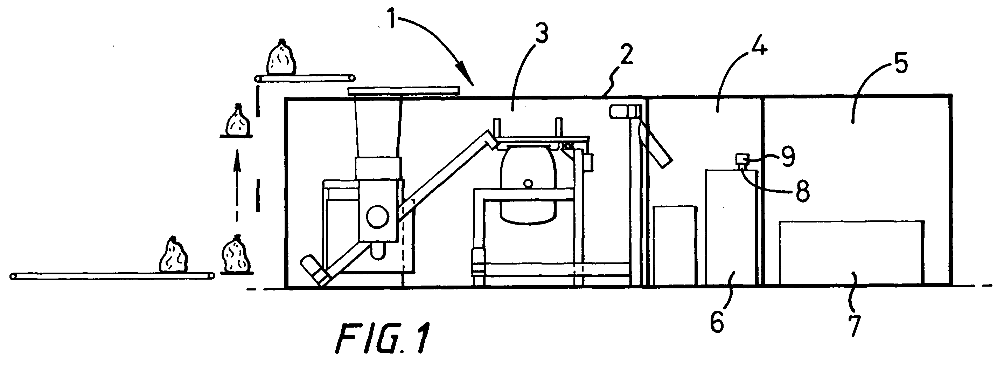

Referring to Figure 1 of the drawings, the apparatus 1 thereshown is largely accommodated in an ISO container 2 for convenience of transport and rapid installation and to provide a chassis for the apparatus. The container is divided into three sections. The first section 3 contains sterilisation apparatus, the second section 4 accommodates a steam generator 6, and the third section 5 houses a control unit 7.

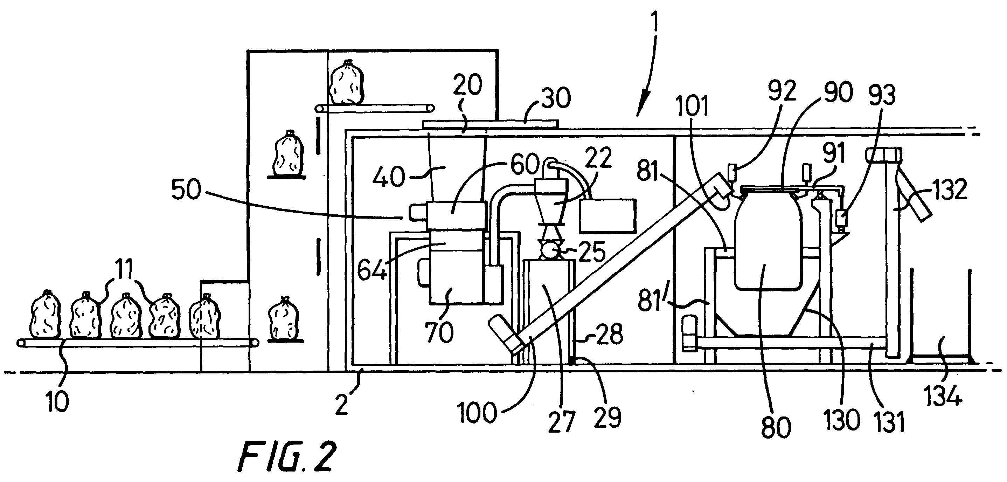

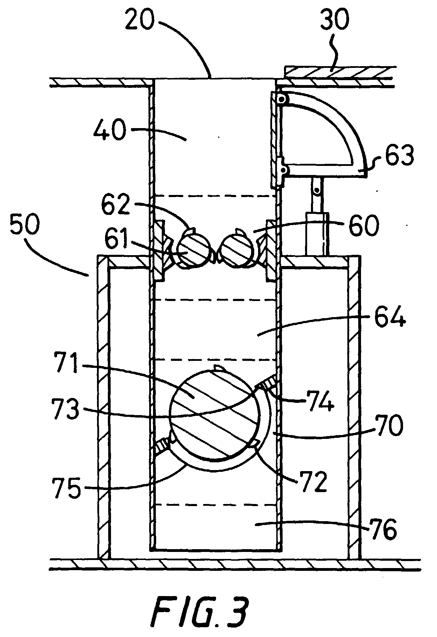

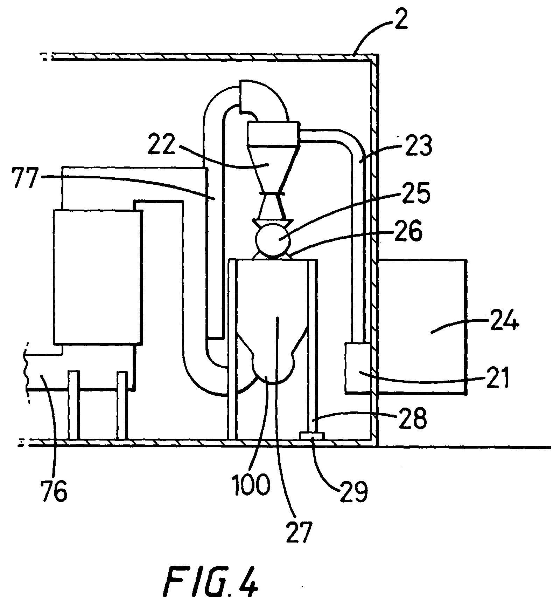

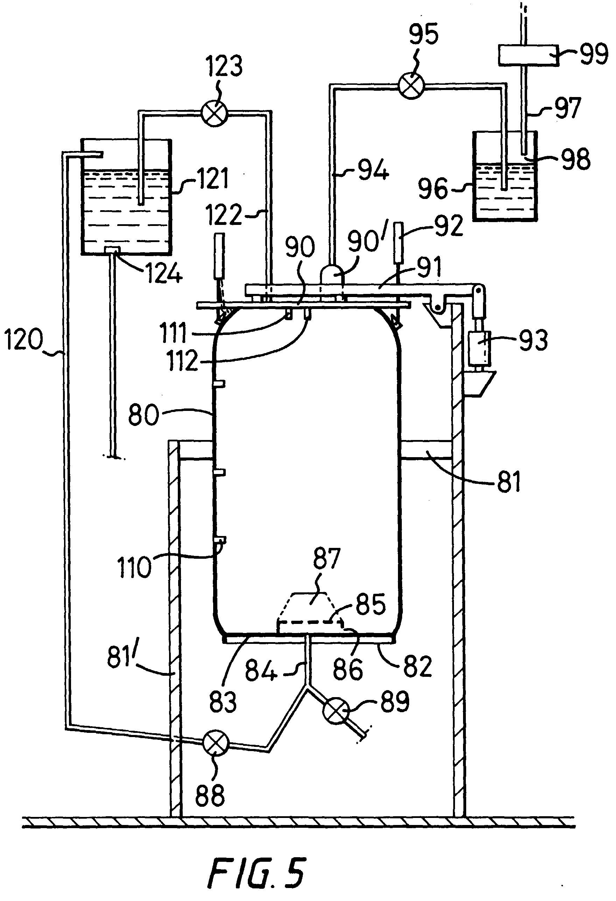

Turning to Figures 2, 3, 4 and 5, for loading waste material into the apparatus 1 a conveyer 10 is provided outside the container 2 and onto which bags of waste material 11 to be processed are placed to be lifted to one at a time over the container to an inlet 20 to the sterilisation apparatus. The conveyor has a capacity to lift 50 bags an hour.

At the inlet 20 in the top of the container a slide valve 30 is provided, with a chute 40 under the valve, leading to a comminution section 50 of the apparatus. A first stage of this section is a shredder 60 having two spindles 61, each carrying along its length several sets of three circumferentially spaced, radially extending blades 62. When the spindles are driven in opposite directions by a motor (not shown), the blades inter-digitate with those on the other spindle for shredding waste passing between them. A pivotally mounted arm 63 is arranged to pass through a side wall of the chute 40 for pushing the waste towards the blades 62 and obviating its bouncing off the blades.

A further chute section 64 leads to a granulator 70. This consists of a drum 71 having three straight, parallel blades 72. The drum is driven at 400 rpm. The blades 72 on the drum 71 co-operate with two slightly curved blades 73 fixedly mounted to a casing 74 on opposite sides of the drum. Immediately below the drum a steel plate 75 having 5mm sieve apertures is arranged. Below the sieve, a comminuted material reception channel 76 is provided. The blades 72, 73 are individually mounted for ease of replacement.

From one end of the channel 76, a duct 77 leads up to the inlet to a cyclone 22. The central air outlet 23 from the cyclone is to a fan 21, which discharges to atmosphere via a HEPA filter 24 and a deodorising filter (not shown) if required. The filters are installed outside the container 2. The fan draws air from the waste material inlet valve 30. This is perforated to allow air into the section 3 when closed. Alternatively, inlet vent(s) can be provided in the wall(s) of the container. Air is also drawn from within the section whereby it is kept below ambient pressure. The air is drawn through the shredder and the granulator in sufficient volume to draw the comminuted material with it through the duct 77 into the cyclone.

The cyclone has a constantly rotating crossed vane valve 25 at its lower outlet 26, allowing the comminuted material to pass down whilst preventing any appreciable upwards air flow. A holding hopper 27 is provided beneath the valve 26. It is of a size to accommodate a full load of 120kg of material. To measure this, it is pivotally supported at one bottom side 28 and supported on load cell(s) 29 on the other bottom side. It is also provided with a vibrator (not shown) to ensure that the comminuted material inside it is evenly distributed.

An auger 100 leads up from the base of the hopper 27, the auger being of a capacity to deliver the 120kg of material in three minutes.

For sterilisation of the comminuted material, the apparatus is provided with a stainless steel pressure vessel 80 of cylindrical shape. The pressure vessel 80 is mounted centrally on a motorised pivot mechanism 81 in a frame 81' fixed in the container 2. This arrangement enables the vessel to be turned upside down. A stainless steel lid 90 is provided for the pressure vessel 80. The lid is pivotally mounted in the frame via a pivot arm 91 and actuator 93 set to one side of the vessel. The lid is provided with twelve pneumatic clamps 92 for locking the lid to a rim of the vessel when upright and with a pair of O-rings (not shown) for pressure tight sealing of the lid to the vessel.

The base 82 of the pressure vessel is strengthened with a 25mm steel plate 83, to which a steam inlet pipe 84 is fitted. Above the inlet pipe 84 is mounted a slotted ring 85 surmounted with a cone 87, having 1mm perforations.

The vessel's lid 90 has a cold dome 90' - for drying steam passing from it - to which is fitted an outlet pipe 94, having a valve 95 and leading to a closed bubbler 96. An outlet pipe 97 leads from the air space 98 at the top of the bubbler through a flow meter 99 to the air space within the section 3 of the container, which is maintained below ambient pressure by the cyclone fan.

The steam generator, which has pressure regulating and safety valves 8, 9, is connected to the steam inlet pipe 84 via a valve 89. It is set up to generate steam at a temperature of 150°C and 4 bar pressure.

In use of the apparatus, under control of the unit 7, the conveyer 10 lifts one bag per minute - the time required to comminute it - to the waste inlet valve 30, which is automatically opened for the bag to fall through to the comminution means 50. As soon as the waste has entered the apparatus the slide valve 30 is closed. The cyclone fan is tun continuously drawing air through the valve and deterring any waste material from passing back out of the apparatus.

In synchronism with the valve 30 being opened for a fresh bag of waste, the arm 63 in the shredder 60 is lifted and returned on top of the bag after a suitable delay to keep it in contact with the shredder. The shredded material falls into the granulator, which like the shredder, is running continuously. The material is chopped up to a size to fall through the sieve 75, any material which is initially too large being picked up and carried around for further chopping between the rotating and fixed blades 72,73. Typically the chopped material will have a major dimension of approximately 3mm.

The chopped or comminuted material is drawn up the duct 77 by the air flow through the cyclone and there separated from the air. It falls into the hopper 27 and is weighed until a load of 120kg has accumulated. The pressure vessel's lid is lifted and the vessel is tilted by the mechanism 81 towards the outlet 101 from the auger, which discharges the hopper into the vessel.

The vessel is returned upright and the lid 90 clamped on 92. Steam is generated and fed via the pipe 84 - with the valve 89 open - to the vessel, where it passes up through the perforated cone 87 and into the waste material. The cone distributes the steam into the load of waste material.

Before steam is fed into the pressure vessel a valve 95 in the outlet pipe 94 is opened. On introduction of steam, the air in the pressure vessel is forced out. The air passes down the pipe 94 and into the bubbler 96. This air passes from the bubbler via the flow meter 99 and thence to the suction side of the fan 21 in the comminution section 50 of the apparatus, where it is discharged to atmosphere via the HEPA filter 24. While air is being expelled from the pressure vessel, it will bubble through the water in the bubbler 96 and a flow rate will be recorded in the outlet pipe 97. However, once all the air has been expelled and steam flows from the pressure vessel, the steam will condense in the water in the bubbler and the flow rate through the outlet pipe will drop dramatically. When this drop in flow rate is recorded, the valve 95 to the bubbler is closed.

Steam continues to enter the pressure vessel from the steam generator. As the steam comes into contact with the cold perforated cone 87 and cold waste material it condenses giving up its latent heat. As more steam enters the pressure vessel, the latter and the contained material are quickly heated. Three temperature probes 110 set at different heights in the pressure vessel measure the temperature of the material. Condensed water, through capillary action, is able to pass quickly through the waste material and convey heat to it. In this way the heat from the steam is rapidly transferred throughout the whole of the pressure vessel and its contents. Another temperature probe 111 and a pressure probe 112 are situated in the lid 90 of the pressure vessel and when all of the probes are giving steady readings the pressure vessel and the steam generator have reached an equilibrium with a steady state being achieved in the pressure vessel. This is the regulated steam pressure of 4 bar and hence the temperature in the vessel is approximately 150°C. At this temperature any bacterial cell and spores will be killed within a period of 2-4 minutes. A lower temperature may be used and the holding time will be correspondingly longer. If the so-call "superbugs" become a problem then a higher temperature may be used and the conditions held for a longer period of time.

After a period of time, a valve 89 is closed to isolate the pressure vessel from the steam generator. By this time a quantity of water will have built up in the pressure vessel, trials suggesting between 10 and 15 litres of water for 120kg of waste material. By opening a valve 88 in a drain pipe 120 from the bottom of the vessel, most of this water can be bled to a second bubbler 121. Slots 86 in the ring 85 enable the water to be separated from the waste material without blocking the pipe 120. As the pressure vessel is still under pressure this water will be rapidly ejected and the valve will need to be opened for a very short period of time. To ensure that only water is bled from the bottom of the pressure vessel, either the valve will be opened for a set period of time, or a set volume of water will be removed. There will usually be a small quantity of water left in the bottom of the pressure vessel.

The steam is then released from the pressure vessel. This is achieved through a second outlet pipe 122 having a valve 123 from the lid 90 of the vessel. This steam outlet pipe 122 leads to the same, second bubbler 121. The latter receives clean water and is mounted in the "clean" section 4 of the container. It is drained after each sterilisation cycle via a drain cock 124. The pressure vessel and its contents will still be hot and the sudden drop in pressure will result in the water left in the pressure vessel including that in the waste material being above its boiling point for the new pressure. Much of this water will therefore evaporate and there will be considerable drying of the waste material.

To remove the waste material from the pressure vessel 80, the lid 90 is unclamped and lifted and the vessel is turned upside down. The waste falls into a holding hopper 130, which is also vibrated. At this stage the waste material is still hot and sticky and to effect its removal from the pressure vessel pressurised air or water may be added through the base of the vessel, (which is now at the top).

While the pressure vessel is still in this position, and an arm (not shown) wipes the rim of the pressure vessel to ensure that it is free from any waste material, and to ensure a good seal for the lid.

The waste material from the hopper is still hot and is conveyed via a horizontal and a vertical screw conveyer 131,132 to a holding bin 134. Both the conveyers 131, 132 have cold water jackets to cool the material.

The waste material is now sterile and can be disposed of in the same manner as any other waste, including landfill.

As the waste material has a high calorific value due to the high quantity of plastics material in the waste, it can be pressed into briquettes or pellets for use in a furnace. The waste material is loaded into a pelletiser and passed through a die, preferably a ½" die. The frictional temperature and the plastic content cause the material to become sticky, so that the material adheres together in briquettes without added adhesive.

The invention and its use is not intended to be restricted to the details of the above described embodiment. In particular it can be used for processing of waste from non-medical establishments which may have a small risk of containing hazardous material.

## Claims

1. A method of sterilising waste material consisting in the steps of: -
• comminuting the waste material;
• loading the comminuted waste material into a pressure vessel and closing the vessel;
• applying steam to the material in the pressure vessel at least until the steam pressure in the vessel is substantially steady, whereby the material is heated to elevated temperature and sterilised; and
• removing the sterilised waste material from the pressure vessel.

2. A method of sterilising waste material as claimed in claim 1, wherein air is substantially expelled from the pressure vessel before it is pressurised with steam.

3. A method of sterilising waste material as claimed in claim 2, wherein steam is flowed through the pressure vessel to expel air from it.

4. A method of sterilising waste material as claimed in claim 3, wherein the steam enters the pressure vessel through it's base and the air is allowed to escape through it's lid.

5. A method of sterilising waste material as claimed in any preceding claim, wherein the steam is applied by ducting it from a steam generator.

6. A method of sterilising waste material as claimed in claim 5, wherein the pressure of the steam is controlled at the steam generator.

7. A method of sterilising waste material as claimed in claim 6, wherein the pressure in the steam generator and the pressure vessel are allowed to reach equilibrium and maintained for a predetermined period thereafter.

8. A method of sterilising waste material as claimed in claim 7, wherein the steady pressure is at least 0.8 bar (corresponding to a temperature of 116°C) and the predetermined period is at least substantially 30 minutes.

9. A method of sterilising waste material as claimed in claim 7, wherein the steady pressure is at least 1.8 bar (corresponding to a temperature of 131.2°C) and the predetermined period is at least substantially 10 minutes.

10. A method of sterilising waste material as claimed in claim 7, wherein the steady pressure is at least 4 bar (corresponding to a temperate of 151.8°C) and the predetermined period is at least substantially 5 minutes.

11. A method of sterilising waste material as claimed in any preceding claim, wherein after sterilisation the steam application is isolated and water is bled out of the pressure vessel.

12. A method of sterilising waste material as claimed in claim 11, wherein the steam pressure is released from the vessel after water drainage, resulting in at least partial drying of the waste material.

13. A method of sterilising waste material as claimed in claim any preceding claim, wherein the sterilised waste material is cooled for disposal.

14. A method of sterilising waste material as claimed in any one of claims 1 to 13, wherein, the sterilised waste material is formed into briquettes or pellets, preferably whilst still hot from sterilisation.

15. Apparatus for use in the sterilisation of waste material, the apparatus comprising in combination: -
• comminution means, for preliminarily comminuting the waste material;
• a pressure vessel for receiving the comminuted waste material to be sterilised, the pressure vessel having:
• a loading aperture via which the comminuted material can be received into the vessel;
• a lid for pressure tightly closing the aperture; and
• steam inlet to the vessel;
• a chassis in which the comminution means and the pressure vessel are mounted;
• a steam generator for supplying steam at a predetermined pressure to the pressure vessel via the steam inlet for sterilising the waste material by application of steam to it.

16. Apparatus as claimed in claim 15, wherein the steam inlet is in the base of the pressure vessel for allowing the steam to percolate upwards through the comminuted waste.

17. Apparatus as claimed in claim 16, wherein a perforated cone is provided at the steam inlet, for distributing application of steam to the comminuted waste.

18. Apparatus as claimed in any one of claims 15 to 17, wherein the pressure vessel is pivotally mounted on the chassis to enable the pressure vessel to be inverted for waste discharge therefrom.

19. Apparatus as claimed in claim 18, wherein the lid is pivotally mounted to the chassis independently from the pressure vessel's pivot to enable the pressure vessel to be inverted while its lid is held in its open position.

20. Apparatus as claimed in any one of claims 15 to 19, wherein the comminution means includes a shredder for shredding sheet material in the waste, a granulator for granulating particulate material in the waste, and a sieve to ensure that waste material with a major diameter greater than a predetermined value is retained in the granulator.

21. Apparatus as claimed any one of claims 15 to 20, wherein the apparatus includes an enclosure on the chassis for the comminution means, an air pump to maintain the enclosure below ambient pressure during comminution and preferably a HEPA filter to remove any airborne contamination before venting.

22. Apparatus as claimed in claim 21, including a duct from the comminution means along which the pump can draw comminuted waste, a cyclone filter for separating the waste from the air flow and a hopper for collecting the separated waste.

23. Apparatus as claimed in claim 22, wherein the apparatus includes a screw conveyer to convey the comminuted waste material from the hopper to the pressure vessel.

24. Apparatus as claimed in any one of claims 15 to 23, wherein the apparatus includes a first bubbler connected to a first outlet in the lid of the pressure vessel, such that as the pressure vessel is filled with steam expelled air bubbles through the bubbler.

25. Apparatus as claimed in claim 24, wherein the apparatus includes means for measuring the flow rate of air out of the first bubbler.

26. Apparatus as claimed in any one of claims 15 to 25, wherein the apparatus includes a second bubbler connected to a second outlet in the pressure vessel, such that as the pressure is released after sterilisation process, the steam is vented into the bubbler.

27. Apparatus as claimed in any one of claims 15 to 26, wherein the apparatus includes a pelletiser for pressing the sterilised waste material into pellets.
